# EUROPÄISCHE PATENTSCHRIFT

(11) **EP 1 344 061 B1**
(45) Veröffentlichungstag und Bekanntmachung des Hinweises auf die Patenterteilung: **02.04.2008**
(21) Anmeldenummer: 01986918.9
(22) Anmeldetag: 20.12.2001
(51) Int. Cl.: G01N 33/543, B01L 3/00

(54) **FESTPHASENSUBSTRATE FÜR STRUKTURIERTE REAKTIONSSUBSTRATE**
SOLID PHASE SUBSTRATES FOR STRUCTURED REACTION SUBSTRATES
SUBSTRATS EN PHASE SOLIDE POUR SUBSTRATS DE REACTION STRUCTURES

(30) Priorität: 20.12.2000 EP 00127996
(43) Veröffentlichungstag der Anmeldung: 17.09.2003
(73) Patentinhaber: Direvo Biotech AG, 50829 Köln (DE)
(72) Erfinder: RARBACH, Markus, Direvo Biotech AG, 50829 Köln (DE); KOLTERMANN, Andre, Direvo Biotech AG, 50829 Köln (DE); KETTLING, Ulrich, Direvo Biotech AG, 50829 Köln (DE)
(74) Vertreter: Hertz, Oliver
(86) Internationale Anmeldenummer: PCT/EP2001/015128
(87) Internationale Veröffentlichungsnummer: WO 2002/050539

(56) Entgegenhaltungen:
- EP-A- 0 408 940
- US-A- 5 141 719
- US-A- 5 223 133
- US-A- 5 227 137
- US-A- 5 858 770

## Beschreibung

Die Erfindung betrifft ein Festphasensubstrat, einen Verbund aus Reaktions- und Festphasensubstraten und Verfahren zur Prozessierung von fluidischen Proben in mindestens einem Reaktionssubstrat.

Strukturierte Reaktionssubstrate (oder: Probenträger) zur Aufnahme und parallelen Manipulation einer Vielzahl von Proben haben große Bedeutung in Biochemie, Medizin und Gentechnik. Neben der Erhöhung der Probenzahl pro Probenträger und Verringerung des Probenvolumens (Miniaturisierung) wird zusätzlich und gleichzeitig eine Erhöhung der Zahl der in den Reaktionssubstraten durchführbaren Manipulationen (Funktionalität) angestrebt. Eine wichtige Untergruppe zu realisierender Funktionen von Reaktionssubstraten sind gekennzeichnet durch Wechselwirkungen der flüssigen Probe mit festen Materialien, die beispielsweise als Festphasensubstrat mit der Probe in einem Reservoir des Reaktionssubstrates in Verbindung gebracht werden. Dieser Gruppe sind verschiedene Funktionen des Festphasensubstrats zuzurechnen, wie beispielsweise die Filtration flüssiger Proben zur Abtrennung partikulärer Probenbestandteile, Reaktionen an festen Oberfläche, wobei die Oberfläche eine an der Reaktion beteiligte Substanz trägt oder aus solchen Substanzen gefertigt wird, sowie physikalische Wechselwirkungen von Probenbestandteilen mit Oberflächen oder an diesen gebundenen Substanzen.

Festphasenfunktionalisierte Reaktionssubstrate insbesondere zur Filtration von Proben sind an sich bekannt. Nachteile dieser bekannten Ausführungen von Reaktionssubstraten im Sinne o. g. Anwendungen ist die Tatsache, dass die Funktionsschicht fest mit dem Reaktionssubstrat verbunden ist. So werden Membranen oder Filter mit einem Reaktionssubstrat unter Begrenzung der Probenkompartimente irreversibel verbunden. Entsprechende Reaktionssubstrate sind beispielsweise in US 4 797 259 beschrieben.

In US 5 009 780 (und entsprechend EP 408 940) sind Reaktionssubstrate beschrieben, in denen Festphasen (Filtermembranen) mit Reaktionssubstraten gekoppelt werden, die die Trennung der Filtermembranen vom Reaktionssubstrat gestatten. Hier sind sowohl eine Isolierung von Filtrat und Retentat als auch eine Bestimmung von Eigenschaften des Filtrats einer Vielzahl von Proben möglich. Das Filtermedium kann aber nur für jedes Probenkompartiment getrennt isoliert werden, so dass eine parallele Verarbeitung nur in der Verbindung des Filtermediums mit dem Reaktionssubstrat möglich ist. Eine Neukombination der Funktionsschicht mit einem anderen Reaktionssubstrat oder die direkte Bestimmung physikochemischer Eigenschaften des an die Funktionsschicht gebundenen Retentats sind nicht möglich. Eine erneute Kopplung der Funktionsschicht mit einem weiteren Reaktionssubstrat ist nicht möglich. Weiter ist der Aufbau des funktionalisierten Reaktionssubstrats komplex, da jedes Probenkompartiment getrennt mit der Festphase verbunden wird. Eine Übertragung des Prinzips aus US 5 009 780 auf Reaktionssubstrate mit einer großen Zahl von Probenkompartimenten (>100 pro Reaktionssubstrat) ist technisch nicht sinnvoll.

In US 4 317 726, US 5 047 215 und US 4 493 815 sind Festphasenelemente reversibel mechanisch zwischen Reaktionssubstraten fixiert. Die Funktionsschicht kann vom Reaktionssubstrat getrennt werden. Die Abdichtung des Filters gegen das Reaktionssubstrat sowie die Abdichtung der Probenkompartimente untereinander erfolgt hier durch mechanischen Druck auf das Festphasenelement durch das Reaktionssubstrat bzw. durch zusätzliche eingefügte Dichtungselemente zwischen Reaktionssubstrat und Festphasenelement (US 4 493 815). Der Dichtungsdruck wird durch Verschraubung des Aufbaus aus Reaktionssubstrat und Festphase erreicht (US 4 317 726, US 4 493 815). Dieser Aufbau bedingt einerseits eine aufwendige Bauweise der Reaktionssubstrate, da die notwendigen Dichtkräfte durch das Reaktionssubstrat aufgenommen werden müssen und behindert die automatisierte Handhabung einer Vielzahl von Probenträgern, da die Trennung von Probenträger und Festphase eine komplexe Handhabung des Reaktionssubstrats erfordert. Da die Dichtkräfte von der Festphase aufgenommen werden müssen, stellen insbesondere bei mechanisch instabilen Membranmaterialien mögliche Beschädigungen der Festphase ein Problem dar. In US5047215 wird die Durchtrennung der Membran durch die Dichtungskräfte zur Trennung der Membrankompartimente benachbarter Probenkompartimente ausgenutzt. Eine Trennung von Festphase und Reaktionssubstrat und eine Neukombination mit anderen Reaktionssubstraten ist damit nicht möglich. Keines der genannten festphasenfunktionalisierten Reaktionssubstrate gestattet die Kopplung mit Substraten anderer Funktionalität. Insbesondere ist die parallele Überführung fluider Proben in Reaktionssubstrate zur spektroskopischen Messung physikochemischer Eigenschaften nicht möglich.

US 5 227 137 und US 5 141 719 beschreiben jeweils Filtrationsapparaturen, die aus einzelnen schicht- oder plattenformigen Komponenten zusammengesetzt sind. Ein fester Zusammenhalt der Komponenten im Betriebszustand wird durch Bildung einer Unterdruckverbindung (US 5 227 137) oder eine Verschraubung (US 5 141 719) bewirkt.

Weitere Nachteile der herkömmlichen Festphasensubstrate bestehen in deren eingeschränkter Verwendbarkeit. So sollten beispielsweise die festen Funktionselemente die Messung physikochemischer Eigenschaften der Probe nicht behindern. Auch kann die Durchführung von komplexen Abfolgen von Manipulationen der Probe notwendig sein. Diese Abfolgen erfordern bisher eine serielle Überführung der Proben in andere Reaktionssubstrate. Solche seriellen Schritte sind sowohl zeitlich als auch apparativ aufwendig.

Die Aufgabe der Erfindung ist es, verbesserte Festphasensubstrate anzugeben, mit denen die Nachteile der herkömmlichen Festphasensubstrate überwunden werden. Die Aufgabe der Erfindung ist es insbesondere, Festphasensubstrate für strukturierte Reaktionssubstrate anzugeben, deren Funktionsschichten frei mit beliebigen Funktionen der Reaktionssubstrate kombinierbar sein sollen, ohne die Verwendungsmöglichkeiten der Reaktionssubstrate selbst oder andere mögliche Manipulationen des Reaktionssubstrat oder der Funktionsschicht einzuschränken. Die Funktionsschicht soll insbesondere vom Reaktionssubstrat ohne Verlust des Retentats oder Filtrats trennbar sein und ggf. mit einem anderen Reaktionssubstrat zu einem funktionalisierten Reaktionssubstrat kombinierbar sein. Die Aufgabe der Erfindung ist es auch, Verfahren zur Herstellung und Verwendung von Festphasensubstraten anzugeben.

Diese Aufgaben werden durch ein Festphasensubstrat, ein funktionalisiertes Reaktionssubstrat und Verfahren mit den Merkmalen der Patentansprüche 1, 9, 13 bzw. 18 gelöst. Vorteilhafte Ausführungsformen und Anwendungen der Erfindung ergeben sich aus den abhängigen Ansprüchen.

Die Grundidee der Erfindung besteht darin, ein Festphasensubstrat anzugeben, das durch eine Festphasenschicht mit mindestens einer Funktionsfläche gebildet wird, wobei auf der Festphasenschicht mindestens eine Verbindungsschicht vorgesehen ist, die aus einem Material besteht, das mit Festkörperoberflächen eine inhärente, lösbare Adhäsionsverbindung eingeht. Anwendungsabhängig ist das Material für eine Adhäsion an Festkörperoberflächen ausgewählt, die aus Polymer-, Kunststoff-, Glas- oder Halbleitermaterialien oder Metallen bestehen. Die Oberfläche der Verbindungsschicht ist auf die jeweilige Festkörperoberfläche abgestimmt. Gemäß einer bevorzugten Ausführungsform der Erfindung ist die Verbindungsschicht eine Polymerschicht, die aus einem Elastomeren mit adhäsiven Eigenschaften gebildet ist. Sie besteht vorzugsweise aus Silikon, insbesondere Polysimethylsiloxan (PDMS), oder auch z. B. aus einem natürlichen oder einem synthetischen Kautschuk, Polyurethanen mit adhäsiven Eigenschaften, Polyisopren oder Acrylelastomeren. Die Verbindungsschicht geht eine Adhäsionsverbindung mit einer glatten (z. B. gegossenen, polierten oder gewalzten) Festkörperoberfläche ein. Die Verwendung der Polymerschicht besitzt den Vorteil, dass das Festphasensubstrat mit beliebigen Substraten, insbesondere Reaktionssubstraten, die die jeweiligen Festkörperoberflächen bereitstellen, kombinierbar ist.

Die Ausstattung der Festphasenschicht mit der adhärierenden Verbindungsschicht besitzt den Vorteil, dass mit einem relativ einfachen, integralen Aufbau das Festphasensubstrat über die Verbindungsschicht für die Dauer einer Probenbehandlung an einem Reaktionssubstrat ohne einen Klebstoff oder eine gesonderte Verbindung stabil fixiert werden und anschließend für weitere Behandlungsschritte insbesondere beschädigungsfrei entfernt und/oder mit einem anderen Reaktionssubstrat verbunden werden kann.

Die Verbindungsschicht ist ein- oder beidseitig auf der Festphasenschicht vorgesehen. Es können mehrere Verbindungsschichten auf der einen oder beiden Seiten der Festphase vorgesehen sein. Eine beidseitige Verbindungsschicht besitzt den Vorteil, dass ein Aufbau aus einem Reaktionssubstrat und einem Festphasensubstrat mit mindestens einem weiteren Reaktionssubstrat und/oder einer Abdeckschicht aus einem zumindest teilweise undurchlässigen Material (z. B. Glas) kombinierbar ist.

Besondere Vorteile ergeben sich, wenn das Festphasensubstrat eine Verbindungsschicht mit einer Vielzahl von Ausnehmungen aufweist, die entsprechend eine Vielzahl von Funktionsflächen der Festphasenschicht freilassen. Die Funktionsflächen sind entsprechend der Anordnung und Form von Reservoiren eines Reaktionssubstrates gebildet. Als Reaktionssubstrat wird allgemein ein Substrat oder Träger mit mindestens einem Probenreservoir bezeichnet, in dem eine fluidische Probe aufgenommen werden kann. Erfindungsgemäß können die Funktionsflächen in Form, Größe und Anordnung gerade der Geometrie der Probenreservoire des Reaktionssubstrats entsprechen. Alternativ kann es von Vorteil sind sein, wenn mehrere Funktionsflächen jeweils einem Probenreservoir oder umgekehrt mehrere Probenreservoire jeweils einer Funktionsfläche zugeordnet sind.

Das Festphasensubstrat ist über die Verbindungsschicht mit dem Reaktionssubstrat verbunden. Diese Ausführungsform der Erfindung ermöglicht vorteilhafterweise, dass in der Verbindung mit dem Reaktionssubstrat eine parallele Verarbeitung einer Vielzahl von Proben und auch eine Neukombination des Festphasensubstrates mit einem anderen Reaktionssubstrat oder eine direkte Messung physikochemischer Eigenschaften an den Funktionsflächen (bzw. an diesen gebundenen Retentaten) möglich sind.

Gegenstand der Erfindung ist auch ein funktionalisiertes Reaktionssubstrat, das einen Verbund aus mindestens einem Reaktionssubstrat und mindestens einem Festphasensubstrat darstellt, die ausschließlich adhäsiv miteinander verbunden sind. Die Verbindung von Festphasen- und Reaktionssubstraten zur Herstellung funktionalisierter Reaktionssubstrate ist vorteilhafterweise einfach und ggf. automatisiert durchführbar. Die Herstellung eines funktionalisierten Reaktionssubstrates erfolgt vorzugsweise derart, dass das Festphasensubstrat durch Andrücken an das Reaktionssubstrat fixiert wird.

Die genannte Aufgabe wird insbesondere durch ein Festphasensubstrat gelöst, auf das in festgelegten Bereichen eine feste, adhäsive, liquophobe Schicht als Verbindungsschicht so aufgebracht ist, dass in einem stapelförmigen Aufbau aus Reaktionssubstraten und Festphasensubstraten in den genannten beschichteten Bereichen eine adhäsive Kopplung zwischen Reaktions- und Festphasensubstrat zustande kommt. Die Beschichtung der Festphasenschicht nimmt dabei vorzugsweise die mit den Probenkompartimenten der verwendeten Reaktionssubstrate korrespondierenden Bereiche der Festphase aus. Gegenstand der Erfindung ist auch ein Verfahren zur Herstellung des Festphasensubstrates. Als Beschichtungsmaterial werden in besonders bevorzugter Ausführungsform Polymere (insbesondere Silikone) verwendet, die in flüssigem Zustand auf die Festphase aufgetragen werden und zu einer festen, flexiblen, adhäsiven Beschichtung polymerisieren. Alternativ kann die Verbindungsschicht auch auf die Festphasenschicht aufgeklebt werden. Die Herstellung der Festphasensubstrate ist technisch relativ einfach, so dass sie vorteilhafterweise Einwegprodukte bilden können.

Zur Herstellung des beschichteten Festphasensubstrats werden in bevorzugter Ausführungsform drucktechnische Verfahren verwendet. In besonders bevorzugter Ausführungsform werden Siebdruckverfahren benutzt, um das Beschichtungsmaterial der Verbindungsschicht gerichtet in Bereichen der Festphase aufzutragen die in kombinierten, funktionalisierten Reaktionssubstraten der Kopplung zwischen Festphasensubstrat und Reaktionssubstrat dienen. Im Siebdruckverfahren sind geeignete strukturierte Beschichtungen mit Auflösungen bis zu einigen µm auf feste Oberflächen aufzubringen. Siebdruckschablonen sind mit lithografischen Verfahren leicht herzustellen und durch Wahl der verwendeten Siebdruckgewebe und Beschichtungen auf die Eigenschaften der zu verarbeitenden Polymere und der zu bedruckenden Materialen abzustimmen.

Die vorzugsweise zur Herstellung des Festphasensubstrates und/oder des Reaktionssubstrates verwendeten Silikonwerkstoffe sind gegenüber chemischen und biochemischen Reaktionsbedingungen weitgehend inert. Eine Vielzahl entsprechender Materialien insbesondere zur Beschichtung von Oberflächen sind dem Fachmann an sich bekannt und verfügbar. Silikone, insbesondere PDMS, bilden mit Festkörperoberflächen nicht-kovalente, adhäsive Bindungen. Die Bindungen zwischen Oberfläche und Silikonsubstrat ist dabei reversibel. Eine Vielzahl von Bindungs-Trennungs-Zyklen ist durchführbar, ohne die Adhäsionseigenschaften des Silikonsubstrats wesentlich zu vermindern. Dies wird z. B. in den unveröffentlichten Patentanmeldungen DE 199 48 087.7 und PCT/EP00/09808 beschrieben.

Die adhäsive Kopplung wahrt in aus den genannten beschichteten Festphasen- und Reaktionssubstraten hergestellten stapelförmigen, festphasenfunktionalisierten Reaktionssubstraten die Integrität aus Festphase und Reaktionssubstrat ohne die Notwendigkeit einer formschlüssigen Verbindung der Bestandteile. Die zusammengesetzten, funktionalisierten Reaktionssubstrate sind als Einheit manuell oder ggf. automatisiert zu handhaben. Gleichzeitig führt die adhäsive Kopplung zwischen Festphasen- und Reaktionssubstrat zur Dichtung der Kopplungsbereiche, so dass sowohl das Austreten flüssiger Probenbestandteile aus dem Reaktionssubstrat als auch das Übertreten von flüssigen Bestandteilen zwischen Reaktionskompartimenten verhindert wird. Das Festphasensubstrat kann ohne Beschädigung sowohl des Festphasensubstrats als auch des Reaktionssubstrats von diesem getrennt werden. Sowohl Reaktionssubstrat als auch Festphase können danach mit anderen Reaktionssubstraten oder Bestandteilen von Reaktionssubstraten kombiniert werden, so dass aus der Neukombination Reaktionssubstrate anderer oder auch gleicher Funktion hervorgehen. Diese reversible und wiederholbare Kopplung von Funktionselementen und Reaktionselementen erlaubt die parallelisierte Durchführung von komplexen Manipulationsfolgen mit einer Vielzahl von Proben.

Vorteilhafterweise können erfindungsgemäße Festphasensubstrate allein d.h. ohne Verbindung mit einem Reaktionssubstrat einer Bestimmung physikochemischer Eigenschaften unterzogen werden, d.h. die Integrität der Probenschicht bleibt auch nach Trennung vom Reaktionssubstrat erhalten.

Durch Wahl des Materials der Festphase können verschiedene Funktionen eines festphasenfunktionalisierten Reaktionssubstrats erreicht werden.

Ein Vorteil der Verbindungsschicht besteht auch darin, dass die Festphasenschicht eines Festphasensubstrats mechanisch stabilisiert und gegen Zerstörungen geschützt wird. Damit werden neue und insbesondere dünnere oder sprödere Festphasenmaterialien, mit denen bislang Festphasenreaktionen nur beschränkt durchführbar waren, der Verwendung zur Prozessierung fluidischer Proben zugänglich.

Weitere Vorteile und Einzelheiten der Erfindung werden im folgenden unter Bezug auf die beigefügten Zeichnungen beschrieben. Es zeigen:
- Figur 1: schematische Darstellungen einer Ausführungsform eines erfindungsgemäßen Festphasensubstrates;
- Figur 2: ein Verfahrensschema zur Verwendung eines erfindungsgemäßen Reaktionssubstrates zur Filtration;
- Figur 3: ein Verfahrensschema zur Verwendung eines erfindungsgemäßen Reaktionssubstrates zur Probenbehandlung;
- Figur 4: ein Verfahrensschema zur Verwendung eines erfindungsgemäßen Reaktionssubstrates zur Durchführung von Festphasenreaktionen;
- Figur 5: eine Lithografievorlage zur Herstellung der Siebdruckschablone für ein erfindungsgemäßes Festphasensubstrat;
- Figur 6: eine Illustration der spektrometrischen Untersuchung erfindungsgemäß behandelter Proben; und
- Figur 7: eine Illustration der spektrometrischen Messung an einem erfindungsgemäß untersuchten Retentat.

Die Erfindung wird im Folgenden unter Bezug auf ein Festphasensubstrat beschrieben, dass zur Kombination mit einem Reaktionssubstrat mit einer Vielzahl von Probenreservoiren eingerichtet ist. Die Erfindung ist jedoch nicht auf Festphasensubstrate mit einer Vielzahl von Funktionsflächen beschränkt, sondern entsprechend auch als Festphasensubstrat mit einer einzelnen Funktionsfläche umsetzbar. Des Weiteren bezieht sich die folgende Erläuterung auf eine bevorzugte Ausführungsform der Erfindung, bei der die Verbindungsschicht auf der Festphasenschicht eines Festphasensubstrates aus Silikon oder anderen Kunststoffen gebildet ist. Das Silikon geht eine Verbindung mit einem Reaktionssubstrat und/oder einer Deckschicht ein, die ebenfalls aus Silikon oder Kunststoff-, Glas- oder Halbleitermaterialien bestehen. Die Erfindung ist analog umsetzbar mit Festphasensubstraten, bei denen die Verbindungsschicht aus Polymer-, chemisch organischem Kunststoff-, Glas- oder Halbleitermaterial oder Metall besteht und eine Adhäsionsverbindung mit dem Reaktionssubstrat und/oder der Abdeckschicht aus Silikon oder einem anderen adhärierenden Material eingeht.

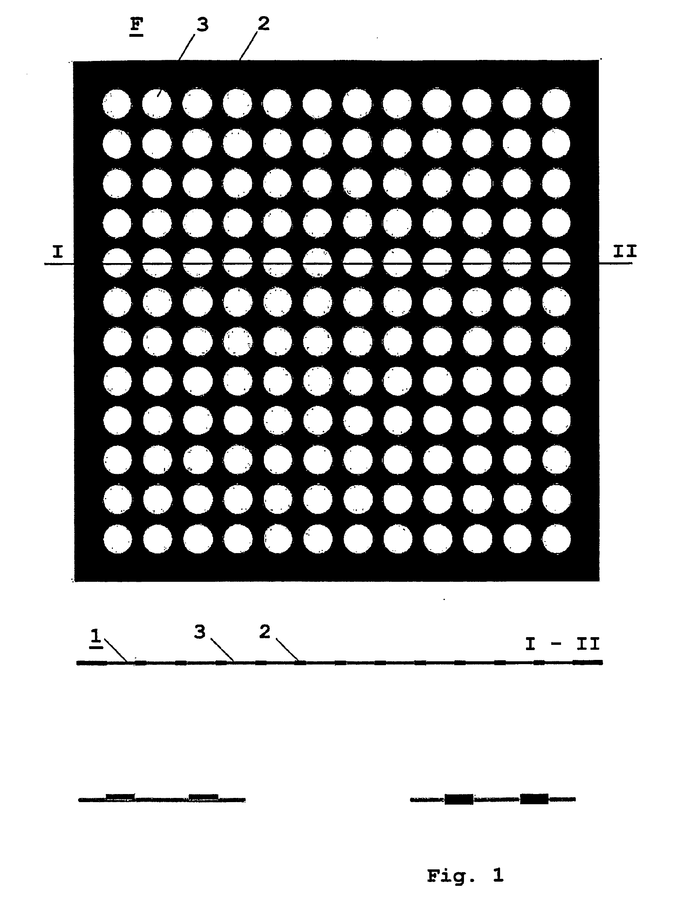

Bei der in Figur 1 in schematischen vergrößerten Drauf- und Seitenansichten illustrierten Ausführungsform eines erfindungsgemäßen Festphasensubstrates F ist auf einer Festphasenschicht 1 (siehe Schnittansicht I-II) eine Verbindungsschicht 2 gebildet. Die Verbindungsschicht 2 ist mit Ausnehmungen strukturiert, so dass die Festphasenschicht 1 an den Funktionsflächen 3 frei liegt. Die Funktionsflächen 3 bilden die gewünschten Festphasen, mit denen fluide Proben in Kontakt gebracht werden sollen. Die Verbindungsschicht 2 kann einseitig (Figur 1, Einschub links unten) oder beidseitig und/oder durchdringend (Figur 1, Einschub rechts unten) auf der Festphasenschicht 1 aufgebracht sein.

Das Festphasensubstrat F bildet vorzugsweise eine ebene, flexible Schicht, kann aber anwendungsabhängig auch gekrümmt entsprechend der Form einer vorbestimmten Bezugsfläche ausgebildet sein. Die Festphasenschicht 1 besteht anwendungsabhängig aus einem Festphasenmaterial, wie es von herkömmlichen Festphasensubstraten an sich bekannt ist. Die Dicke der Festphasenschicht liegt bspw. im Bereich von ca. 1 µm bis 10 mm, vorzugsweise im Bereich von ca. 100 µm bis 1 mm. Die Flächenmaße und -formen des Festphasensubstrates F und der Funktionsflächen 3 sind anwendungsabhängig entsprechend den Dimensionen eines Reaktionssubstrates und der in diesem gebildeten Reservoire für fluide Proben gewählt. Das in Figur 1 dargestellte Festphasensubstrat F besitzt bspw. 144 Funktionsflächen 3 in geraden Reihen und Spalten angeordnet. Erfindungsgemäße Festphasensubstrate stellen vorteilhafterweise keine Beschränkungen in Bezug auf die Flächenmaße der Funktionsflächen dar.

### Herstellung des Festphasensubstrats

Wird ein poröses Festphasenmaterial verwendet, so wird in bevorzugter Ausführungsform der Erfindung das liquophobe Beschichtungsmaterial und das Beschichtungsverfahren so gewählt, dass im Prozess der Beschichtung das Beschichtungsmaterial die poröse Festphase durchdringt (Figur 1: Schnittansicht I-II).

Der Beschichtungsprozess des Festphasenmaterials mit dem adhäsiven, liquophoben Beschichtungsmaterial kann nur von einer Seite des Materials (Figur 1: links unten) oder in deckungsgleicher Wahl der beschichteten Bereiche von beiden Seiten des Festphasenmaterials (Figur 1: rechts unten) erfolgen. Das Festphasensubstrat kann damit gleichzeitig mit zwei Reaktionssubstraten oder Bestandteilen von Reaktionssubstraten gekoppelt werden.

Die beidseitige Kopplung eines Festphasensubstrats mit zwei Reaktionssubstraten erlaubt die parallele Prozessierung und Überführung von flüssigen Probenbestandteilen von einem Reaktionssubstrat in ein anderes. Die beiden Substrate können dabei an sich gleiche oder unterschiedliche Funktionalitäten aufweisen.

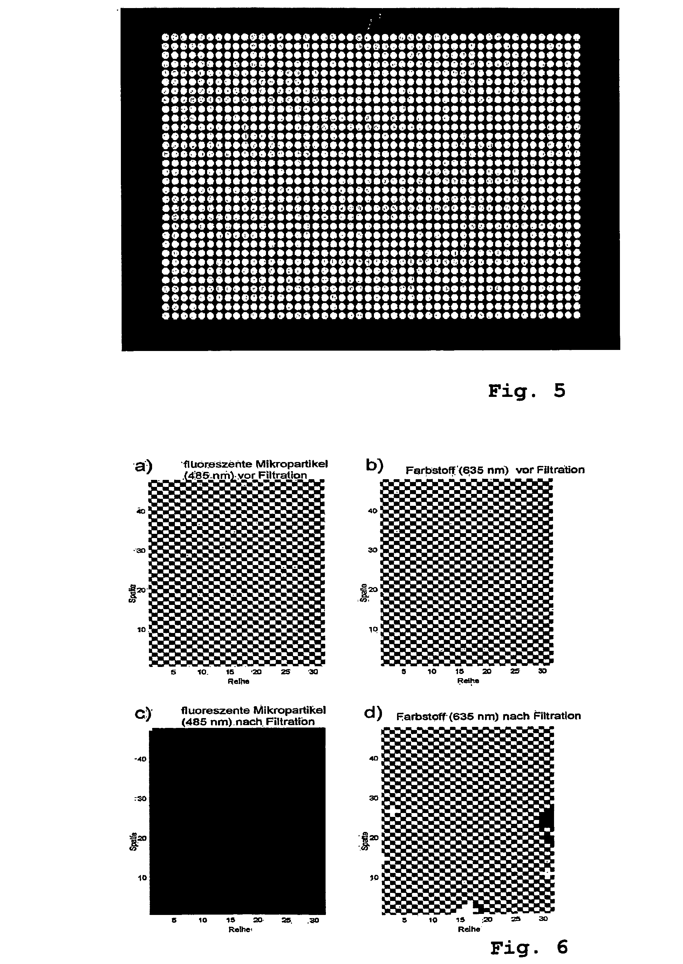

Als Beschichtungsverfahren zur Herstellung der Verbindungsschicht 2 auf der Festphasenschicht 1 werden vorzugsweise drucktechnische Verfahren verwendet. Druckvorlagen für unterschiedliche Reaktionssubstrate können erstellt und lithografisch auf Siebdruckgewebe übertragen werden. Die verwendeten Siebdruckgewebe können nach dem Fachmann bekannten Vorgehensweisen auf die chemischen und rheologischen Eigenschaften des zu verarbeitenden Polymers abgestimmt werden. Zur Herstellung eines Festphasensubstrates für ein Reaktionssubstrat mit 1536 Probenreservoiren (Probenkompartimente, siehe Figur 5) wird bspw. ein Siebdruckgewebe (27 Faden/cm) mit lösungsmittelresistenter Fotolackbeschichtung (50 µm Schichtdicke) verwendet. Die Siebdruckschablone wird mit der in Figur 5 dargestellten Lithographiemaske belichtet (Herstellung der Siebdruckschablone durch das Unternehmen "Werbung & Druck", Göttingen).

Als Beschichtungspolymer wird ein handelsübliches Produkt verwendet (Wacker Finish CT 51 L, Wacker Chemie GmbH, München). Das gewählte Polymer wird nach Herstellerangaben vorbereitet und weist eine Topfzeit von mehreren Stunden auf (Herstellerangaben). Die Polymerisation des Materials erfolgt nach jedem Beschichtungsvorgang durch Inkubation des beschichteten Festphasenelements bei einer Temperatur von 100°C für 2 Minuten.

Die Festphasenschicht besteht bspw. aus einem Filtrationsmaterial, das durch Celluloseacetatmembran der Porengröße 1,2 µm (Schleicher und Schüll, Produkt.Nr. ST 69) gebildet wird. Das Filtrationsmaterial wird durch Klebung in den nicht zu bedruckenden Randbereichen der Filtrationsmembran auf einer glatten, festen Unterlage fixiert. Das fluide Polymer wird mit der Siebdruckschablone auf das Festphasenmaterial aufgebracht. Jeder Beschichtungsschritt ist gefolgt von einem Polymerisationsschritt. Beidseitige Beschichtung des Festphasensubstrats erfordert deckungsgleiches Aufbringen der Polymerschichten in beiden Beschichtungsvorgängen. Die Positionierung von Siebdruckschablone und Festphasensubstrat kann nach Aufbringen von Kontrollmarkierungen nach dem ersten Beschichtungsvorgang visuell kontrolliert werden.

Das so hergestellte Festphasensubstrat wird kombiniert mit Silikon-Reaktionssubstraten, deren Verteilung der Reaktionskompartimente der in Figur 5 wiedergegeben Siebdruckvorlage des Festphasenelements entspricht. Entsprechende Reaktionssubstrate werden bspw. in der unveröffentlichten Anmeldung PCT/EP00/09808 (DE 199 48 087.7) beschrieben. Das 4 mm starke Silikon-Reaktionssubstrat nimmt 1536 Probenkompartimente auf und kann an der unteren und/oder oberen Peripherie durch eine ebene, mit dem Reaktionssubstrat adhäsiv verbundenen Glasplatte abgeschlossen werden.

Figur 1 zeigt als wichtiges Merkmal der Erfindung, dass jede Funktionsfläche in der Substratebene vollständig von der Verbindungsschicht und/oder einem in die Festphasenschicht eingedrungenen Teil der Verbindungsschicht umgeben wird. Benachbarte Funktionsflächen sind relativ zueinander isoliert. Ein Flüssigkeitskontakt (Kontamination) zwischen benachbarten Festphasen ist ausgeschlossen. Der lateral flüssigkeitsdichte Einschluss der Funktionsflächen erfordert jedoch nicht notwendig die Bildung einer geschlossenen Verbindungsschicht.

Im Folgenden werden verschiedene Verfahrensweisen bei der Verwendung erfindungsgemäßer Festphasen- und Reaktionssubstrate erläutert.

### Filtration

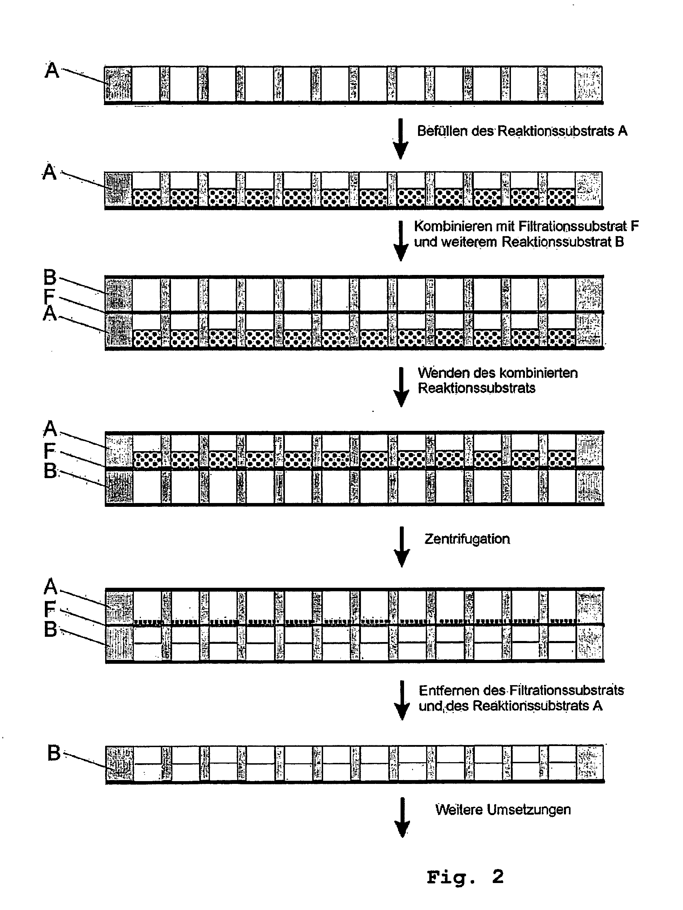

Figur 2 zeigt ein Verfahrensschema zur Illustration der Verwendung eines erfindungsgemäßen Aufbau aus einem ersten Reaktionssubstrat A und einem Festphasensubstrat F in Kombination mit einem weiteren Reaktionssubstrat B.

Zur Filtration von Proben können als Festphasenschicht Filtrationsmedien mit der genannten adhäsiven Verbindungsschicht versehen werden. Als Filtrationsmedien werden in bevorzugter Ausführungsform Filtrationsgewebe oder -papiere mit nominellen Porengrößen zwischen 10 µm und 500 µm oder Glasfaserfilter mit nominellen Porengrößen zwischen 1 µm und 100 µm verwendet, in weiterer besonders bevorzugter Ausführungsform werden poröse Filtrationsmembranen mit nominellen Porengrößen zwischen 0.1 µm und 10 µm, insbesondere mit Porengrößen zwischen 0.1 µm und 2 µm verwendet. Gemäß einer alternativen bevorzugten Ausführungsform können Ultrafiltrationsmembranen mit einer nominellen Ausschlussgröße zwischen 1 nm und 100 nm verwendet werden. Alle genannten Materialien sind dem Fachmann an sich bekannt und verfügbar.

Das beidseitig mit der liquophoben Beschichtung versehene Festphasensubstrat F wird mit dem ersten strukturierten Reaktionssubstrat A, dessen Probenkompartimente die fluiden Proben trägt, adhäsiv gekoppelt. Die Kopplung erfolgt bspw. durch Auflegen des Festphasensubstrates F auf das Reaktionssubstrat A, so dass die Reaktionsflächen und die Probenreservoire zueinander ausgerichtet sind. Anschließend wird das Festphasensubstrat F manuell oder mit einer Anpasseinrichtung (z. B. mit einem Stempel mit einer Größe entsprechend der Fläche des Reaktionssubstrates) angedrückt, so dass die Adhäsionsverbindung zwischen beiden Teilen gebildet wird. Auf das mit dem ersten Reaktionssubstrat A verbundene Festphasensubstrat F wird eine zweites Reaktionssubstrat B aufgelegt, so dass sich das Festphasensubstrat F in einem stapelförmigen Aufbau zwischen dem ersten Reaktionssubstrat A und dem zweiten Reaktionssubstrat B befindet. Nach Wenden des zusammengesetzten Reaktionssubstrats A-F-B können die fluiden Proben durch Zentrifugation vom ersten in das zweite Reaktionssubstrat überführt werden. Filtrat und Retentat werden bei diesem Schritt getrennt. Das Filtrat kann im Reaktionssubstrat B isoliert werden, während das Retentat am Festphasensubstrat F bzw. im kombinierten Reaktionssubstrat aus Reaktionssubstrat A und Festphasensubstrat F verbleibt. Sofern das Reaktionssubstrat (Probenträger) B entsprechende Funktionalität besitzt, kann das Filtrat sofort oder nach Entfernen von Reaktionssubstrat A und Festphasenelement F einer Bestimmung physikochemischer Eigenschaften zugeführt werden oder fluide Proben oder das mit dem Retentat beladene Festphasensubstrat F können ggf. nach Kombination mit anderen Reaktionssubstraten oder Teilen von Reaktionssubstraten weiteren Umsetzungen/Prozessschritten unterzogen werden.

### Diffusive Prozesse

Gemäß einer weiteren Ausführungsform der Erfindung werden funktionalisierte Reaktionssubstrate bereitgestellt, die diffusive Transportprozesse durch feste Reaktionselemente ermöglichen. Solche als Festphasen vorgesehene Reaktionselemente können beispielweise aus Werkstoffen hergestellt werden, die den selektiven Austausch von Substanzen auf Grund bestimmter physikochemischer Eigenschaften wie beispielsweise Molekülgröße, Ladung oder hydrophoben/hydrophilen Eigenschaften oder Kombinationen aus diesen Eigenschaften selektiv ermöglichen oder verhindern.

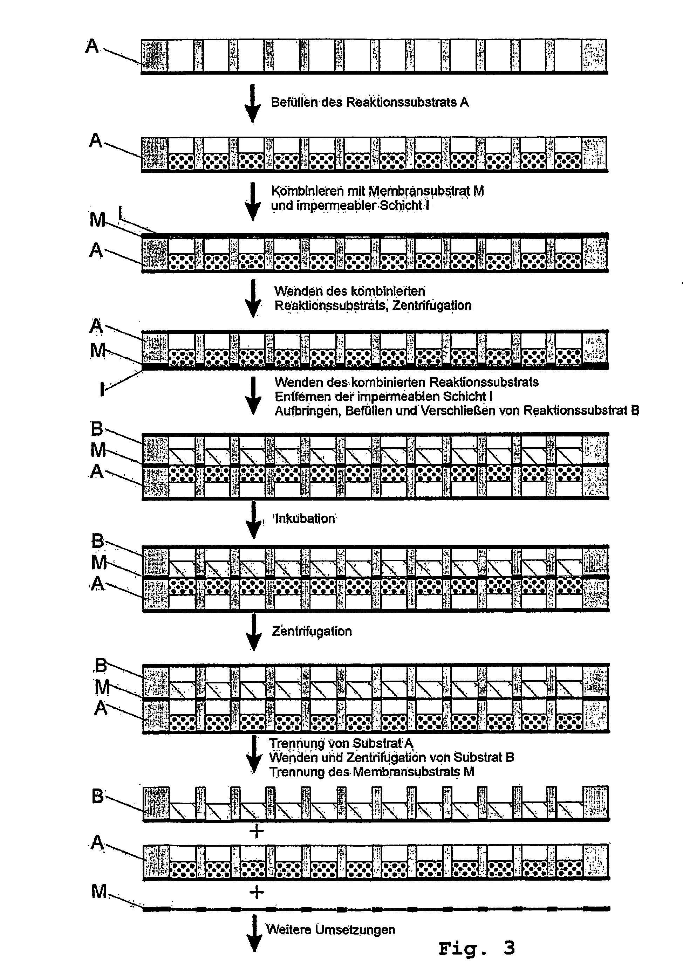

Eine Verfahrensweise unter Verwendung eines Festphasensubstrates, bei dem die Funktionsflächen die genannten Reaktionselemente bilden, ist schematisch in Figur 3 illustriert. Figur 3 zeigt die Verwendung eines Aufbaus aus einem ersten Reaktionssubstrat A mit einem Festphasensubstrat, das durch ein Membransubstrat M gebildet wird, in Kombination mit einem zweiten Reaktionssubstrat B oder einer Abdeckschicht I. Die Funktionsflächen des Membransubstrates (Reaktionselemente) sind bspw. semipermeable Membranen mit Ausschlussgrenzen zwischen 1 nm und 100 nm. Diese Membranen gestatten den selektiven Austausch niedermolekularer Substanzen zwischen fluiden Proben.

Insbesondere in kleinen Probenkompartimenten haften fluide Proben aufgrund der Oberflächenspannung des Probenfluids auch nach Wenden des Reaktionssubstrats an der oberen festen Begrenzung eines Probenkompartiments, so dass in der in Figur 3 dargestellten Ausführungsform fluide Proben in den zwei Reaktionssubstraten A und B über das Membransubstrat M in Kontakt gebracht werden können. Der selektive Stofftransport zwischen den fluiden Proben kann dabei sowohl durch Konzentrationsgradienten zwischen den Proben als auch durch äußere Kräfte wie z.B. Gradienten eines externen elektrischen Feldes bedingt sein. Zur Herstellung eines entsprechend funktionalisierten Reaktionssubstrats gemäß Figur 3 wird ein Reaktionssubstrat A mit den fluiden Proben befüllt, mit dem Membransubstrat M kombiniert und mit der impermeablen Abdeckschicht I verschlossen. Durch Wenden des Substrats und Kombination mit dem zweiten Reaktionssubstrat B entsteht ein kombiniertes Reaktionssubstrat, in dem eine Vielzahl fluider Proben in den Reaktionssubstraten A und B über das Membransubstrat M in Kontakt gebracht werden können. Durch Zentrifugation und Trennen des kombinierten Reaktionssubstrats können die fluiden Proben nach Beendigung des Stoffaustauschs in den genannten Reaktionssubstraten A und B voneinander getrennt werden.

### Reaktion

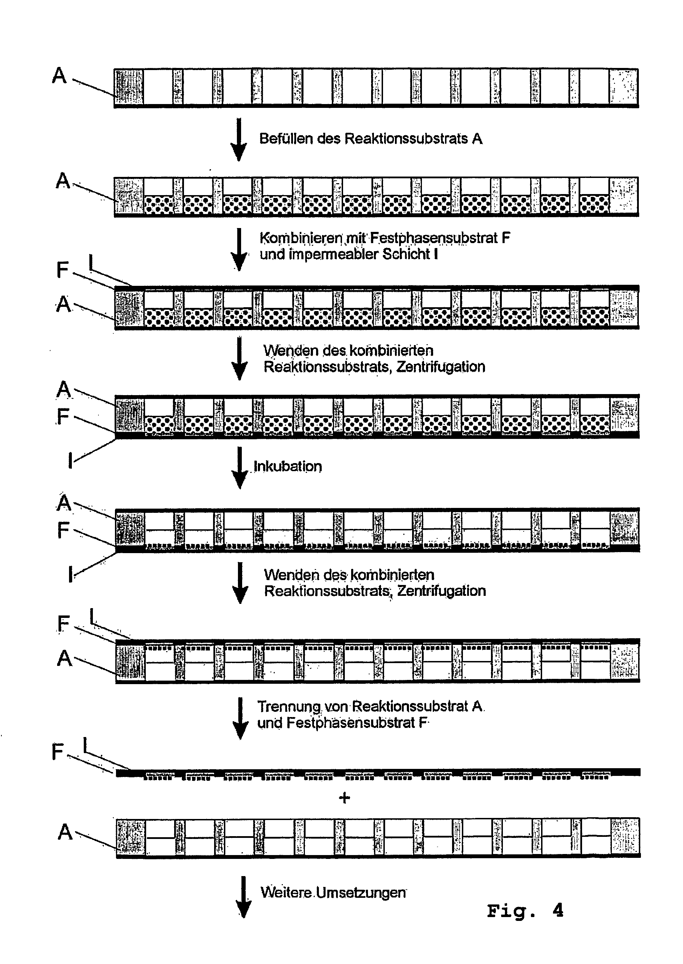

Figur 4 zeigt ein Verfahrensschema zur Verwendung von Festphasensubstraten und funktionalisierter, kombinierter Reaktionssubstrate daraus in Ausführungsformen, die eine Adsorption, Desorption oder Reaktion von Substanzen mit festen Oberflächen ermöglichen. Wiederum ist ein Reaktionssubstrat A mit einem Festphasensubstrat F vorgesehen, die mit der impermeablen Schicht I abgedeckt ist.

Zur Reaktion einer flüssigen Probe oder von Partikeln, die in einer flüssigen Probe suspendiert sind, an einer festen Oberfläche oder mit einer an eine Oberfläche gebundenen Substanz kann eine schicht- oder membranartig ausgeführte reaktive Festphase einseitig oder beidseitig mit der genannten adhäsiven Verbindungsschicht vorgesehen sein. Eine einseitige Beschichtung ist im Allgemeinen dann ausreichend, wenn die reaktive Festphase selbst impermeabel für den fluiden Reaktionspartner oder für Bestandteile der fluiden Proben ist. Ist die reaktive Festphase selbst permeabel für den fluiden Reaktionspartner oder für Bestandteile der fluiden Proben, kann die Festphase beidseitig mit der genannten adhäsiven Beschichtungen versehen werden. Wie in Figur 4 dargestellt ist, können in dieser Ausführungsform die mit den fluiden Proben befüllten Reaktionskompartimente des Reaktionssubstrats A nach Aufbringen der beschichteten, reaktiven Festphase F und ggf. einer Schicht eines impermeablen, an der Reaktion nicht beteiligten Materials I abgeschlossen werden.

Durch Wenden (ggf. zusätzlich durch Zentrifugation) der kombinierten Reaktionssubstrate kann die fluide Probe mit der Festphase in Kontakt gebracht werden bzw. nach der Reaktion von dieser getrennt werden. Nach Beendigung der Umsetzung können die fluiden Proben im Reaktionssubstrat A und das Festphasensubstrat F voneinander getrennt werden und beide einer Bestimmung physikochemischer Eigenschaften zugeführt werden, oder Proben oder Festphasensubstrat können weiteren Umsetzungen/Prozessschritten unterzogen werden.

### Bindung

Analog kann eine Bindung von Bestandteilen einer flüssigen Probe an einer festen Oberfläche oder mit einer an eine Oberfläche gebundenen Substanz erfolgen, wobei eine schicht- oder membranartig ausgeführte adsorptive Festphase einseitig oder beidseitig mit der adhäsiven Verbindungsschicht versehen wird. Eine einseitige Beschichtung ist dann ausreichend, wenn die adsorptive Festphase selbst impermeabel für den fluiden Reaktionspartner oder Bestandteile der fluiden Proben ist. Ist die adsorptive Festphase selbst permeabel für den fluiden Reaktionspartner oder Bestandteile der fluiden Proben, kann die Festphase beidseitig mit der genannten adhäsiven Beschichtungen versehen werden. Die Reaktionskompartimente können dann nach Aufbringen der beschichteten, reaktiven Festphase mit einem impermeablen, an der Reaktion nicht beteiligten Material abgeschlossen werden. Wie in Figur 4 dargestellt, kann die adsorptive Festphase F mit einem mit fluiden Proben befüllten Reaktionssubstrat A kombiniert werden.

Durch Wenden (ggf. zusätzlich durch Zentrifugation) des funktionalisierten Reaktionssubstrats kann die fluide Probe mit der Festphase in Kontakt gebracht werden bzw. nach der Reaktion von dieser getrennt werden. Nach der Umsetzung können Reaktionssubstrat A und Festphasensubstrat F voneinander getrennt werden und beide einer Bestimmung physikochemischer Eigenschaften zugeführt werden, oder Proben oder Festphasensubstrat können weiteren Umsetzungen/Prozessschritten unterzogen werden. In gleicher Weise können funktionalisierte Reaktionssubstrate hergestellt werden, deren Festphasenfunktion in der Desorption einer festphasengebundenen Substanz durch Wirkung einer fluiden Probe oder Bestandteilen der fluiden Probe besteht. Die Adsorption/Desorption von Substanzen selbst kann dabei sowohl durch Bestimmung der physikochemischen Eigenschaften der fluiden Probe als auch der festphasengebundenen Probenbestandteile beobachtet werden.

Da die Festphasensubstrate eine wiederholte Kopplung an Reaktionssubstrate gestatten, können die beschriebenen Ausführungsformen sowohl konsekutiv in einer festgelegten Manipulationsfolge durchgeführt werden als auch durch gekoppelte Manipulation der fluiden Proben in einem Verfahrensschritt realisiert werden. In einer besonders bevorzugten Ausführungsform kann ein funktionalisiertes Reaktionssubstrat zur reaktiven Umsetzung eines festphasengebundene Reaktanden mit nachfolgender Desorption von Reaktionsprodukten in die fluide Probe hergestellt werden. Wird ein Reaktionssubstrat geeigneter Funktionalität verwendet, ist die nachfolgende Bestimmung physikochemischer Eigenschaften der Probe möglich. In weiterer bevorzugter Ausführungsform können auch Reaktions- und Filtrationsfunktionalitäten des Festphasensubstrats gekoppelt werden, in dem reaktive Bestandteile an ein geeignetes poröses Filtrationsmaterial gebunden werden. So können ggf. an der Reaktion beteiligte partikuläre Probenbestandteile nach Beendigung der Reaktion abgetrennt werden. Diese Funktionskombination kann wünschenswert sein, da partikulären Bestandteile die Bestimmung physikochemischer Eigenschaften der Probe behindern können, oder da die Reaktion durch Abtrennung der partikulären Feststoffe gestoppt werden muss.

### Messergebnisse

Die hervorragenden Filtrationseigenschaften des erfindungsgemäßen Festphasensubstrats werden durch die im Folgenden erläuterten Fluoreszenzmessungen bestätigt. Die Messungen erfolgen mit einer Suspension von fluoreszierenden Mikropartikeln (Fluospheres 505/515, Durchmesser 2 µm, Molecular Probes, Eugene, OR, USA) in einer Fluoreszenzfarbstofflösung (Cy5-Succinimidyl-ester, Molecular Probes, Eugene, OR, USA). Farbstoff und Partikel können fluoreszenzspektrometrisch auf Grund verschiedener Absorptions- und Emissionseigenschaften unterschieden werden. Während die Stoffmenge der verwendeten Partikel durch die Fluoreszenzemission im Wellenlängenbereich 530-560 nm bei einer Anregungswellenlänge von 485 nm quantifiziert werden kann, wird die Stoffmenge des verwendeten Farbstoffs durch die Fluoreszenzemission im Wellenlängenbereich von 665-735 nm bei einer Anregungswellenlänge von 635 nm bestimmt. Zur Bestimmung der Fluoreszenzeigenschaften der Proben wird eine Mikrotiterplatten-Reader verwendet (SpectraFluor, TECAN, AT).

In alternierender Abfolge ("Schachbrettmuster") werden die Reaktionskompartimente eines einseitig mit einer Glasplatte verschlossenen Reaktionssubstrats (vgl. Figur 6) mit jeweils 1,3 µl Wasser (Reinstwasser, HPLC-grade) bzw. der oben genannten Suspension fluoreszenter Mikropartikel befüllt. Die Zusammensetzung der fluiden Proben wird wie beschrieben vor und nach der Filtration fluoreszenzspektrometrisch bestimmt.

Das funktionalisierte Reaktionssubstrat wird wie beschrieben und in Figur 2 wiedergegeben aus dem mit den fluiden Proben befüllten ersten Reaktionssubstrat A, dem hergestellten Filtrationssubstrat F und einem weiteren, mit dem ersten Reaktionssubstrat identischen Reaktionssubstrat B hergestellt. Filtrat und Retentat werden durch Zentrifugation getrennt (Hereaus Megafuge 1.0, Schwenkbecherrotor für Mikrotiterplatten Nr. 7586, 2800 rpm, 30 min). Nach Beendigung der Zentrifugation werden die Reaktionssubstrate A und B vom Filtrationssubstrat F getrennt. Die Probenfiltrate in Reaktionssubstrat B und die am Filtrationssubstrat F anhaftenden Probenretentate werden der fluoreszenzspektrometrischen Bestimmung zugeführt.

Die Ergebnisse der fluoreszenzspektrometrischen Messungen der Partikelsuspension vor der Zentrifugation sowie die Messungen des Filtrat nach der Zentrifugation sind in Figur 6 wiedergegeben, wobei die Teilbilder a die Fluoreszenzemission der suspendierten Partikel vor Zentrifugation, b die Fluoreszenzemission des gelösten Farbstoffs vor Zentrifugation, c die Fluoreszenzemission der suspendierten Partikel nach Zentrifugation und d die Fluoreszenzemission des gelösten Farbstoffs nach Zentrifugation zeigen.

Die Messungen der fluiden Proben vor und nach der Zentrifugation wurden unter identischen Messbedingungen durchgeführt. Für die Unterscheidung des Fluoreszenzsignals von Partikelsuspension und Kontrollproben (Wasser) wird in allen Messungen eine Schwellenwert von 5000 (Zählwerte) counts festgelegt. Probenkompartimente mit einer Fluoreszenzemission über dem Schwellenwert sind weiß dargestellt, Probenkompartimente mit einer Fluoreszenzemission unter dem Schwellenwert sind schwarz dargestellt Die Messungen der eingesetzten Proben in Figur 6a und b zeigen alternierende Fluoreszenzsignale entsprechende dem gewählten Dispensierschema ("Schachbrettmuster"). Die Fluoreszenzmessungen der Proben nach Filtration in Figur 6c und d zeigen die Absenkung des auf die eingesetzten fluoreszenten Mikropartikel zurückzuführenden Fluoreszenzsignals (Figur 6c) unter den Schwellenwert und damit die Entfernung der eingesetzten Partikel aus der Probensuspension durch das Filtrationssubstrat sowie die im wesentlichen unveränderten Fluoreszenzsignale des vom verwendeten Filtrationsmedium nicht zurückgehaltenen gelösten Fluoreszenzfarbstoffs im Filtrat.

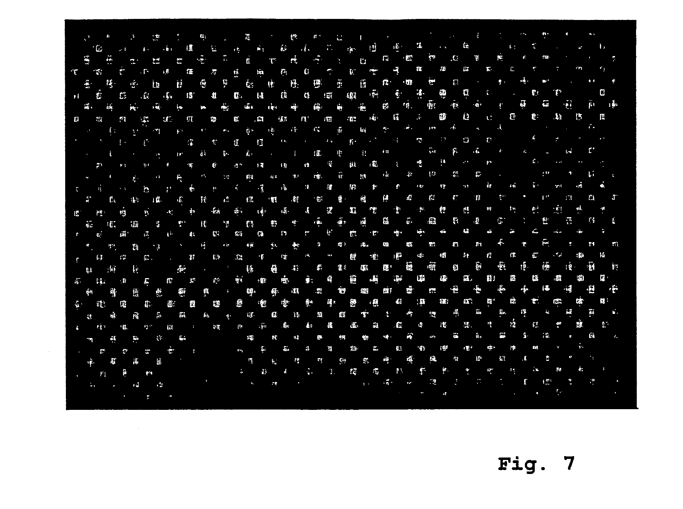

Fluoreszenzspektroskopische Untersuchung (Anregungswellenlänge 485 nm Fluoreszenzemission im Wellenlängenbereich 530-560 nm) des aus dem funktionalisierten Reaktionssubstrat isolierten Filtrationssubstrats F zeigt die Emissionssignale des am Filtrationselement haftenden Retentats (siehe Figur 7). Die bestimmte Fluoreszenzverteilung entspricht dem Dispensierschema der Probensuspension.

Die in der vorstehenden Beschreibung, den Zeichnungen und den Ansprüchen offenbarten Merkmale der Erfindung können sowohl einzeln als auch in beliebiger Kombination für die Verwirklichung der Erfindung in ihren verschiedenen Ausgestaltungen von Bedeutung sein.

## Patentansprüche

1. Festphasensubstrat (F, M) zur adhäsiven Kopplung mit mindestens einem Reaktionssubstrat, wobei das Festphasensubstrat (F, M) eine Festphasenschicht (1) aufweist,
**gekennzeichnet durch**
mindestens eine Verbindungsschicht (2), die auf der Festphasenschicht (1) gebildet und mit dieser fest verbunden ist, wobei die Verbindungsschicht (2) aus einem Material besteht, das mit Oberflächen von Polymer-, Kunststoff-, Glas- oder Halbleitermaterialien oder Metall eine Adhäsionsverbindung eingeht, und mindestens eine Funktionsfläche (3) der Festphasenschicht (1) derart ausnimmt, dass die Funktionsfläche (3) lateral vollständig von der Verbindungsschicht (2) umgeben wird.

2. Festphasensubstrat gemäß Anspruch 1, das sich entsprechend einer vorbestimmten, insbesondere ebenen, Bezugsfläche erstreckt, wobei die mindestens eine Funktionsfläche (3) in Richtung der Ausdehnung der Bezugsfläche allseits von der Verbindungsschicht (2) umgrenzt wird.

3. Festphasensubstrat gemäß einem der vorhergehenden Ansprüche, bei dem die Festphasenschicht (1) eine Filterschicht, eine Reaktivschicht, eine semipermeable Schicht oder eine Adsorptionsschicht ist.

4. Festphasensubstrat gemäß einem der vorhergehenden Ansprüche, bei dem die Verbindungsschicht (2) die Festphasenschicht (1) zumindest teilweise ein- oder beidseitig bedeckt.

5. Festphasensubstrat gemäß einem der vorhergehenden Ansprüche, bei dem die Verbindungsschicht (2) aus Silikon, insbesondere PDMS, besteht.

6. Festphasensubstrat gemäß einem der vorhergehenden Ansprüche, bei dem die Verbindungsschicht (2) aus einem Polymermaterial besteht, das auf der Festphasenschicht (1) diese zumindest teilweise durchdringend angeordnet ist.

7. Funktionales Reaktionssubstrat, bestehend aus mindestens einem Reaktionssubstrat mit mindestens einem Probenreservoir und einem Festphasensubstrat (F, M) gemäß einem der vorhergehenden Ansprüche.

8. Funktionales Reaktionssubstrat gemäß Anspruch 7, bei dem jede Funktionsfläche (3) jeweils ein Probenreservoir abdeckt.

9. Funktionales Reaktionssubstrat gemäß Anspruch 7 oder 8, das einen Verbund aus einem ersten Reaktionssubstrat (A) mit dem Festphasensubstrat (F, M) und einem zweiten Reaktionssubstrat (B) oder einer Abdeckschicht (I) bildet.

10. Funktionales Reaktionssubstrat gemäß Anspruch 9, bei dem das Festphasensubstrat (F, M) auf dem ersten Reaktionssubstrat (A) angeordnet und auf dem Festphasensubstrat (F, M) das zweite Reaktionssubstrat (B) derart angeordnet ist, dass das mindestens eine Probenreservoir des zweiten Reaktionssubstrats (B) zur Funktionsfläche (3) des Festphasensubstrats (F, M) hin geöffnet ist.

11. Verfahren zur Prozessierung von fluidischen Proben durch Wechselwirkung der Proben mit einem Festphasensubstrat (F, M) in einem funktionalen Reaktionssubstrat gemäß einem der Ansprüche 7 bis 10, bei dem am Festphasensubstrat (F, M) eine Filtration, eine chemische Reaktion, insbesondere eine Bindungsreaktion, eine physikalische Wechselwirkung, insbesondere eine Adsorption oder Desorption, und/oder eine substanzselektive Diffusion erfolgen.

12. Verfahren gemäß Anspruch 11, bei dem nach der Prozessierung der Proben das Festphasensubstrat (F, M) oder eines oder beide der Reaktionssubstraten (A, B) voneinander getrennt und separat weiteren Behandlungs- oder Messschritten unterzogen werden.

13. Verfahren gemäß Anspruch 12, bei dem das Festphasensubstrat (F, M) oder eines oder beide der Reaktionssubstraten (A, B), die voneinander getrennt sind, bei den weiteren Behandlungs- oder Messschritten mit weiteren Reaktionssubstraten (A, B) gemäß einem der Ansprüche 7 bis 10 kombiniert werden.

14. Verfahren zur Herstellung eines Festphasensubstrates gemäß einem der Ansprüche 1 bis 6, bei dem das Material der Verbindungsschicht (2) auf die Festphasenschicht (1) im flüssigen Zustand entsprechend der gewünschten Form und Anordnung der mindestens einen Funktionsfläche (3) aufgebracht und durch Polymerisation, Trocknung und/oder Härtung, ggf. mit nachträglicher Sinterung und/oder Verschmelzung, mit der Festphasenschicht (1) verbunden wird.

15. Verfahren gemäß Anspruch 14, bei dem die Verbindungsschicht (2) mit einem Siebdruckverfahren aufgebracht wird.

## Claims

1. Solid phase substrate (F, M) for adhesive coupling with at least one reaction substrate, the solid phase substrate (F, M) having a solid phase layer (1),
**characterised by**
at least one connecting layer (2), which is formed on the solid phase layer (1) and tightly bound therewith, wherein the connecting layer (2) consists of a material which enters into an adhesive bonding with surfaces of polymer, plastics, glass or semiconductor materials or metal, and excludes at least one functional area (3) of the solid phase layer (1) in such a manner that the functional area (3) laterally is completely surrounded by the connecting layer (2).

2. Solid phase substrate according to claim 1 which extends in accordance with a predetermined, in particular planar, reference surface, wherein the at least one functional area (3) is bounded on all sides in the direction of the extent of the reference surface by the connecting layer (2).

3. Solid phase substrate according to any one of the preceding claims, in which the solid phase layer (1) is a filter layer, a reactive layer, a semipermeable layer or an adsorption layer.

4. Solid phase substrate according to any one of the preceding claims, in which the connecting layer (2) covers the solid phase layer (1) at least in part on one or both sides.

5. Solid phase substrate according to any one of the preceding claims, in which the connecting layer (2) consists of silicone, in particular PDMS.

6. Solid phase substrate according to any one of the preceding claims, in which the connecting layer (2) consists of a polymer material which is arranged on the solid phase layer (1) at least partially penetrating the latter.

7. Functional reaction substrate consisting of at least one reaction substrate comprising at least one sample reservoir and a solid phase substrate (F, M) according to any one of the preceding claims.

8. Functional reaction substrate according to claim 7, in which each functional area (3) in each case covers a sample reservoir.

9. Functional reaction substrate according to claim 7 or 8, which forms a composite of a first reaction substrate (A) with the solid phase substrate (F, M) and a second reaction substrate (B) or a cover layer (I).

10. Functional reaction substrate according to claim 9, in which the solid phase substrate (F, M) is arranged on the first reaction substrate (A) and the second reaction substrate (B) is arranged on the solid phase substrate (F, M) in such a manner that the at least one sample reservoir of the second reaction substrate (B) is open towards the functional area (3) of the solid phase substrate (F, M).

11. Method for processing fluid samples by interaction of the samples with a solid phase substrate (F, M) in a functional reaction substrate according to any one of claims 7 to 10, in which filtration, a chemical reaction, in particular a binding reaction, a physical interaction, in particular adsorption or desorption, and/or substance-selective diffusion proceed at the solid phase substrate (F, M).

12. Method according to claim 11, in which, after processing of the samples, the solid phase substrate (F, M) or one or both of the reaction substrates (A, B) are separated from one another and are separately subjected to further treatment or measurement steps.

13. Method according to claim 12, in which the solid phase substrate (F, M) or one or both of the reaction substrates (A, B), which are separate from one another, are combined during the further treatment or measurement steps with further reaction substrates (A, B) according to any one of claims 7 to 10.

14. Method for producing a solid phase substrate according to any one of claims 1 to 6, in which the material of the connecting layer (2) is applied onto the solid phase layer (1) in the liquid state in accordance with the desired shape and arrangement of the at least one functional area (3) and is bonded with the solid phase layer (1) by polymerisation, drying and/or curing, optionally with subsequent sintering and/or fusion.

15. Method according to claim 14, in which the connecting layer (2) is applied by a screen printing method.

## Revendications

1. Substrat en phase solide (F, M) destiné au couplage adhésif avec au moins un substrat de réaction, le substrat en phase solide (F, M) présentant une couche de phase solide (1),
**caractérisé par**
au moins une couche de liaison (2), qui est formée sur la couche de phase solide (1) et reliée solidement à celle-ci, la couche de liaison (2) se composant d'un matériau, qui comprend un composé d'adhésion avec des surfaces de matériaux de type polymère, plastique, verre ou semi-conducteur ou d'un métal, et au moins une surface fonctionnelle (3) se distinguant de la couche de phase solide (1), de manière à ce que la surface fonctionnelle (3) est latéralement complètement entourée par la couche de liaison (2).

2. Substrat en phase solide selon la revendication 1, qui s'étend conformément à une surface de référence prédéterminée, en particulier plane, la surface fonctionnelle (3) au moins au nombre de une étant, dans la direction du développement de la surface de référence, délimitée de tous les cotés par la couche de liaison (2).

3. Substrat en phase solide selon l'une quelconque des revendications précédentes, pour lequel la couche de phase solide (1) est une couche filtre, une couche réactive, une couche semi-perméable ou une couche d'adsorption.

4. Substrat en phase solide selon l'une quelconque des revendications précédentes, pour lequel la couche de liaison (2) recouvre la couche de phase solide (1) au moins partiellement d'un ou des deux côtés.

5. Substrat en phase solide selon l'une quelconque des revendications précédentes, pour lequel la couche de liaison (2) se compose de silicone, en particulier de PDMS.

6. Substrat en phase solide selon l'une quelconque des revendications précédentes, pour lequel la couche de liaison (2) se compose d'un matériau polymère, qui est disposé sur la couche de phase solide (1), traversant au moins partiellement celle-ci.

7. Substrat de réaction fonctionnel, se composant d'au moins un substrat de réaction avec au moins une réserve d'échantillon et un substrat en phase solide (F, M) selon l'une quelconque des revendications précédentes.

8. Substrat de réaction fonctionnel selon la revendication 7, pour lequel chaque surface fonctionnelle (3) recouvre respectivement une réserve d'échantillon.

9. Substrat de réaction fonctionnel selon la revendication 7 ou 8, qui forme un assemblage à partir d'un premier substrat de réaction (A) avec un substrat en phase solide (F, M) et un second substrat de réaction (B) ou une couche de recouvrement (I).

10. Substrat de réaction fonctionnel selon la revendication 9, pour lequel le substrat en phase solide (F, M) est disposé sur le premier substrat de réaction (A) et le second substrat de réaction (B) est disposé sur le substrat en phase solide (F, M), de manière à ce qu'au moins une réserve d'échantillon du second substrat de réaction (B) soit ouverte vers la surface fonctionnelle (3) du substrat en phase solide (F, M).

11. Procédé pour traiter des échantillons fluides par interaction des échantillons avec un substrat en phase solide (F, M) dans un substrat de réaction fonctionnel selon l'une quelconque des revendications 7 à 10, dans le cadre duquel sont effectuées sur le substrat en phase solide (F, M) une filtration, une réaction chimique, en particulier une réaction de couplage, une interaction physique, en particulier une adsorption ou une désorption, et/ou une diffusion sélective selon la substance.

12. Procédé selon la revendication 11, dans le cadre duquel, après le traitement de l'échantillon, le substrat en phase solide (F, M) ou un ou les deux substrats de réaction (A, B) sont séparés l'un de l'autre et sont soumis séparément à des étapes supplémentaires de traitement ou de mesure.

13. Procédé selon la revendication 12, dans le cadre duquel le substrat en phase solide (F, M) ou un ou les deux substrats de réaction (A, B), qui sont séparés l'un de l'autre, sont combinés, lors des étapes supplémentaires de traitement ou de mesure, à d'autres substrats de réaction (A, B) selon l'une quelconque des revendications 7 à 10.

14. Procédé pour la fabrication d'un substrat en phase solide selon l'une quelconque des revendications 1 à 6, dans le cadre duquel le matériau de la couche de liaison (2) est appliqué sur la couche de phase solide (1) à l'état liquide conformément à la forme et à la disposition désirées de la surface fonctionnelle (3) au moins au nombre de une et est relié à la couche de phase solide (1) par polymérisation, séchage et/ou durcissement, éventuellement avec un frittage et/ou une fusion ultérieurs.

15. Procédé selon la revendication 14, dans le cadre duquel la couche de liaison (2) est appliquée par un procédé de sérigraphie.
